# EUROPEAN PATENT APPLICATION

(11) **EP 2 668 907 A2**
(43) Date of publication of application: **04.12.2013**
(21) Application number: 13170118.7
(22) Date of filing: 31.05.2013
(51) Int. Cl.: A61B 17/00

(54) **Specimen retrieval device**

(30) Priority: 01.06.2012 US 201261654212 P; 14.05.2013 US 201313893598
(71) Applicant: Covidien LP, Mansfield, MA 02048 (US)
(72) Inventor: Hathaway, Peter, Lebanon, Connecticut 06249 (US); Sniffin, Kevin, Danbury, Connecticut 06810 (US); Taylor, Eric, East Hampton, Connecticut 06424 (US)
(74) Representative: Maschio, Antonio

(57) **Abstract**

A specimen retrieval device (600) includes a handle (601), a shaft (603) connected to said handle, and a bag deployment device (605) connected to a distal end of the shaft. A specimen retrieval bag (607) is connected to the bag deployment device and includes at least one material having a mesh (608) disposed therein. At least one strand of the mesh is disposed at an angle (609) relative to the bag deployment device and the flexibility of the bag is a function of the angle.

## Description

### CROSS REFERENCE TO RELATED APPLICATION

The present application claims the benefit of and priority to U.S. Provisional Application Serial No. 61/654,212, filed on June 1, 2012, the entire contents of which are incorporated herein by reference.

### BACKGROUND

### 1. Technical Field

The present disclosure relates to a surgical containment apparatus and methods for use thereof. More particularly, the present disclosure relates to a specimen retrieval pouch and method for use in minimally invasive surgical procedures.

### 2. Background of the Related Art

Laparoscopic and endoscopic surgical procedures are minimally invasive procedures in which operations are carried out within the body by using elongated instruments inserted through small entrance openings in the body. The initial opening in the body tissue to allow passage of the endoscopic or laparoscopic instruments to the interior of the body may be a natural passageway of the body, or it can be created by a tissue piercing instrument such as a trocar. Laparoscopic and endoscopic procedures generally require that any instrumentation inserted in the body be sealed, i.e. provisions must be made to ensure that gases do not enter or exit the body through the instrument or the entrance incision so that the surgical region of the body, e.g. the peritoneum, may be insufflated. Mechanical actuation of such instruments is for the most part constrained to the movement of the various components along a longitudinal axis with structure provided to convert longitudinal movement to lateral movement where necessary.

Because the endoscopic or laparoscopic tubes, instrumentation, and any required punctures or incisions are relatively narrow, endoscopic or laparoscopic surgery is less invasive as compared to conventional surgical procedures in which the surgeon is required to cut open large areas of body tissue. Therefore, laparoscopic or endoscopic surgery minimizes trauma to the patient and reduces patient recovery time.

Minimally invasive procedures may be used for partial or total removal of body tissue or organs from the interior of the body, e.g. nephrectomy, cholecystectomy, and other such procedures. During such procedures, it is common that a cyst, tumor, or other affected tissue or organ must be removed via the access opening in the skin, or through a cannula. Various types of entrapment devices have been disclosed to facilitate this procedure.

For example, U.S. Pat. No. 5,037,379 to Clayman et al. discloses a surgical tissue bag for percutaneously debulking tissue by morcellation. The bag includes a layer of puncture-resistant material, a layer of moisture-resistant material and a drawstring. In a disclosed method of use, the bag is placed within the body cavity, the body tissue or organ is placed within the bag, the opening of the bag is pulled through the incision in the skin leaving the distal end of the bag containing the tissue or organ within the body cavity, a morcellator is then inserted into the bag, and then the tissue or organ is debulked and suctioned out of the bag.

U.S. Pat. No. 5,074,867 to Wilk discloses a planar membrane having filaments attached to its corners. The membrane is placed within a body cavity with the filaments extending through the trocar cannula to the outside of the body. The organ or tissue to be removed is placed on the membrane and the filaments are pulled to close the membrane around the organ and draw it through the cannula, if the organ is sufficiently deformable. If the organ is not sufficiently deformable, e.g. because of the presence of gallstones, a forceps or other instrument is used to crush the stones or tissue.

### SUMMARY

As shown in the drawings and described throughout the following description, as is traditional when referring to relative positioning on a surgical instrument, the term "proximal" refers to the end of the apparatus that is closer to the user and the term "distal" refers to the end of the apparatus that is farther away from the user. The term "clinician" refers to any medical professional (e.g., doctor, surgeon, nurse, or the like) performing a medical procedure involving the use of embodiments described herein. As used herein with reference to the present disclosure, the terms "laparoscopic" and "endoscopic" are interchangeable and refer to instruments having a relatively narrow operating portion for insertion into a cannula or a small incision in the skin, or to a surgical procedure in which such instruments are employed. Use herein of the term "laparoscopic" should not be construed to exclude "endoscopic" and use herein of the term "endoscopic" should not be construed to exclude "laparoscopic." To the contrary, it is believed that the present disclosure may find use in any procedure where access to the interior of the body is limited to a relatively small incision, with or without the use of a cannula, including, but not limited to, laparoscopic procedures. The term "about" is to mean ±5% of the described value.

In at least one aspect, the present disclosure is directed towards an apparatus for removing body tissue from the interior of the body as part of a minimally invasive surgical procedure. The apparatus includes a pouch assembly and a pouch support. The pouch assembly preferably includes a pouch and a pouch support. The pouch has an openable end and an opposed closed end. The pouch support can be attached to an applicator assembly. The pouch may have perforations to facilitate detachment of the pouch from the pouch support. The detachment can be simultaneous with the closing of the pouch in response to pulling a drawstring.

In another embodiment, the pouch assembly includes a number of sacks where each sack has a different diameter forming a staggered arrangement of the sacks. Each sack has a mouth at one end. One of the sacks, preferably the most distal sack, has a closed end thereby forming a cavity therein, while the other sacks have an orifice opposite the mouth of the sack. The pouch assembly may have a scored line on one of the sacks to facilitate detachment of the pouch assembly from the support. The pouch assembly can have a scored line extending circumferentially therearound between the locations of the spring structure and the drawstring. The pouch support can be attached to the applicator assembly. The detachment can be simultaneous with the closing of the pouch assembly in response to pulling the drawstring thread.

In some embodiments, the sacks can be fabricated from a material selected from the group consisting of polyurethane and latex and preferably is transparent. One or more reinforced regions or bands extend circumferentially about the pouch assembly and overlap the junction between a pair of adjacent sacks.

The pouch assembly of either embodiment may include a seal formed from an elastic material that is disposed in a proximal region of the pouch assembly, preferably near the mouth. The seal is biased by the elastic material to a closed configuration and is held in an open configuration by the spring. In preferred configurations, the closed configuration of the seal forms a substantially fluid-tight barrier about the external surface of surgical instrument such as the suction tube.

The apparatus can further include structure for resiliently opening the openable end of the pouch assembly, such as spring structure circumferentially attached to the openable end of the pouch assembly and movable between an elongated and narrow closed configuration and a rounded open configuration, the spring structure being resiliently biased to the open configuration. The spring structure, which can support the pouch assembly as well as open it, is attached to the distal end of a drive rod and is slidably movable through a tubular portion of the applicator apparatus when in the closed configuration, and resiliently moveable to its open configuration when moved outside said tubular portion. The spring structure can include two elastic prongs each having a proximal end portion having a side surface in facing relation to the side surface of the proximal end portion of the other elastic prong and fastened thereto, and each elastic prong further having a distal end portion joined to the distal end portion of the other prong by a flexible membrane, such as shrink-wrap type tubing, attached to both said end portions.

The apparatus preferably further includes at least one gaseous sealing structure, such as a coating of viscous sealing material applied to the outer surfaces of the drive rod and the drawstring.

A suction apparatus may be included as part of the removal apparatus. The suction apparatus includes a suction tube, a tubular member, and a suction source. The suction tube is configured and dimensioned to be inserted through an access device such as a trocar cannula.

One preferred method for debulking the tissue specimen is the use of the suction apparatus in combination with any of the previously disclosed embodiments of the pouch assembly. The suction is communicated from the suction source through the tubular member, the suction tube, and ultimately to the pouch assembly through the trocar cannula. By applying a controlled amount of suction, the suction apparatus reduces the volume of solid and/or liquid matter in the pouch assembly. After the desired amount of volume reduction, the applied suction may be reduced or eliminated. The size of the tissue specimen, the volume of liquid present in the pouch assembly, the size of the access opening, and the procedure being performed are considerations in determining the amount of suction applied from the suction apparatus. The flexible membrane of the pouch assembly collapses to conform to the tissue specimen thereby facilitating the removal of the pouch assembly and tissue specimen from the body cavity. By using this method in conjunction with the disclosed embodiments of the pouch assemblies, larger tissue specimens may be removed through trocar cannulae than with conventional pouch assemblies. The suction apparatus may further include storage structure for retaining solid and/or liquid matter removed from the pouch assembly.

In accordance with at least one aspect of this disclosure, a specimen retrieval device includes a handle, a shaft connected to the handle and extending distally, a bag deployment device connected to a distal end of the shaft, and a specimen retrieval bag connected to the bag deployment device and including at least one material having a mesh disposed therein, at least one strand of the mesh being disposed at an angle relative to the bag deployment device, wherein the angle is acute, wherein the flexibility of the bag is selectable as a function of the angle.

In accordance with another aspect of this disclosure the angle may be about 45 degrees.

In accordance with yet another aspect of this disclosure, the angle may be between about 1 and about 45 degrees.

In accordance with still yet another aspect of this disclosure, the material may be at least one fabric including the mesh disposed therein.

In accordance with still yet another aspect of this disclosure, the at least one fabric may be rip-stop nylon.

In accordance with still yet another aspect of this disclosure, a method for modifying flexibility of a specimen retrieval bag includes providing a specimen retrieval device having a handle, a shaft connected to said handle and extending distally, a bag deployment device connected to a distal end of the shaft, and a specimen retrieval bag connected to the bag deployment device and including at least one material having a mesh disposed therein, at least one strand of the mesh being disposed at an angle relative to the bag deployment device, wherein the flexibility of the bag is selectable as a function of the angle, and selecting the angle from a range of about 90 degrees to about 45 degrees to provide a desired flexibility of the specimen retrieval bag between a range of flexibilities, the range of flexibilities being from a minimum flexibility wherein the angle is about 90 degrees to a maximum flexibility wherein the angle is about 45 degrees.

In accordance with still yet another aspect of this disclosure, the angle may be selected to be about 45 degrees for maximum flexibility of the specimen retrieval bag.

In accordance with still yet another aspect of this disclosure, the angle may be selected to be about 30 degrees.

In accordance with still yet another aspect of this disclosure, the angle may be selected to be about 20 degrees.

In accordance with still yet another aspect of this disclosure, the material is rip-stop nylon and the angle is selected to be about 45 degrees for maximum flexibility of the specimen retrieval bag.

In accordance with still yet another aspect of this disclosure, a specimen retrieval device including a handle, a shaft connected to said handle and extending distally, a bag deployment device connected to a distal end of the shaft, and a specimen retrieval bag connected to the bag deployment device and including rip-stop nylon having a rip-stop mesh disposed therein, the rip-stop mesh being disposed at a 45 degree angle relative to the bag deployment device.

### BRIEF DESCRIPTION OF THE DRAWINGS

The above and other aspects, features, and advantages of the present disclosure will become more apparent in light of the following detailed description when taken in conjunction with the accompanying drawings, wherein like reference numerals may refer to similar or identical elements throughout the description of the figures:

FIG. 1A is a perspective view of a pouch assembly and a deployment apparatus according to an embodiment of the present disclosure;

FIG. 1B is a perspective view of a pouch assembly and the deployment apparatus according to another embodiment of the present disclosure;

FIG. 1C is a perspective view of the apparatus in the initial, undeployed configuration;

FIG. 2 is an elevational partially cut away view of the pouch assembly according to a first embodiment of the present disclosure;

FIG. 3 is an elevational partially cut away view of the pouch assembly according to a second embodiment of the present disclosure;

FIG. 3A is another embodiment of the pouch assembly of FIG. 3 having reinforced regions;

FIG. 3B is an alternate embodiment of the pouch assembly of FIG. 3A;

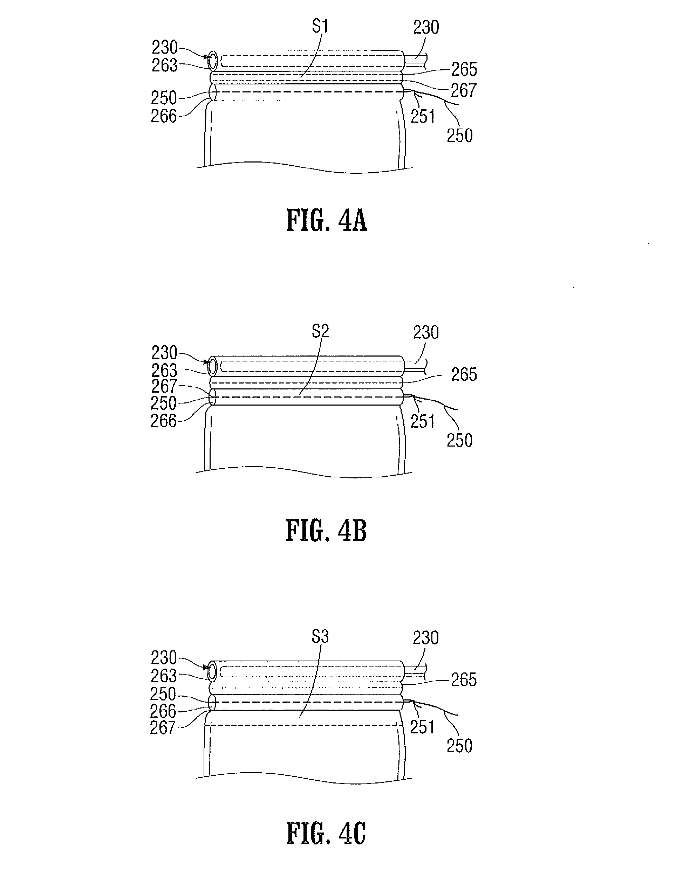

FIGS. 4A-C are elevational partially cut away views of a portion of the pouch assembly with a seal according to embodiments of the present disclosure;

FIGS. 5A-D illustrate a method of minimizing the contents of a pouch assembly in accordance with an embodiment of the present disclosure;

FIG. 6A is a perspective view of at least one embodiment of specimen retrieval device in accordance with the present disclosure;

FIG. 6B is a perspective view of the distal end of a specimen retrieval device of Fig. 6A; and

FIG. 6C is a side view of a portion of the distal end of the specimen retrieval device of Fig. 6A.

### DETAILED DESCRIPTION

Particular embodiments of the present disclosure are described hereinbelow with reference to the accompanying drawings; however, the disclosed embodiments are merely examples of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

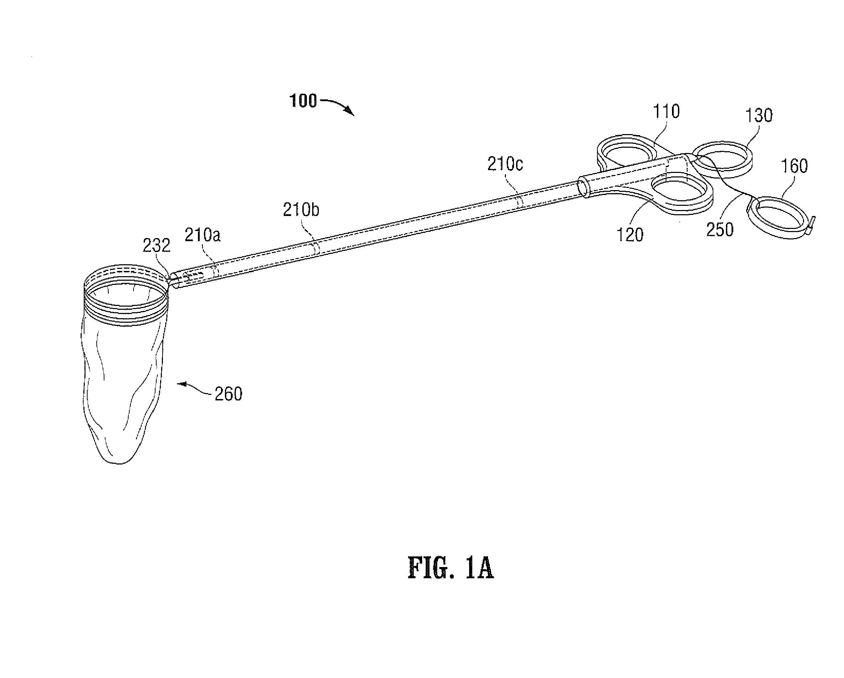

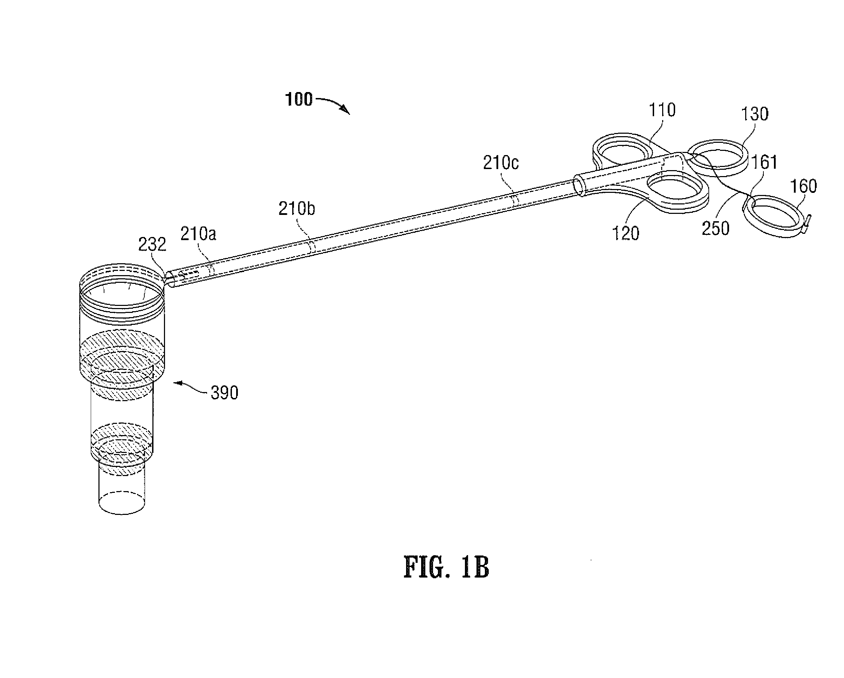

An applicator assembly 100 is illustrated in FIGS. 1A-C. As shown in FIG. 1A, the applicator assembly 100 includes a first embodiment of a removal pouch assembly 260, while FIG. 1B illustrates the applicator assembly 100 with a second embodiment of a removal pouch assembly 390. An applicator assembly suitable for use in conjunction with either removal pouch assembly is disclosed in U.S. Patent No. 5,647,372 to Tovey et al. and in U.S. Patent No. 5,465,731 to Bell et al. and the entire contents of each is hereby incorporated by reference in their entirety.

In some embodiments, the applicator assembly 100 includes an elongated tube 180 which is of such dimensions to be insertable through an access device, such as a trocar cannula, for endoscopic or laparoscopic procedures. The tube 180 is of such diameter as to permit it to be slidably disposed through a trocar cannula for use in endoscopic or laparoscopic operations, and is generally between about 0.25 inches to 0.50 inches in diameter, and about 10 inches to about 15 inches long, although other dimensions may also be used if appropriate to the operation being performed. Tube 180 slidably houses the drive rod 190 and, when undeployed, a spring 230 and pouch assembly 260. In the initial, unused condition, pouch assembly 260 will be rolled up and the spring, including spring portions 231 and 232, will be relatively straight and positioned within tube 180. When the drive rod 190 is advanced, the spring 230 connected thereto will exit the distal end of tube 180 and resiliently pop open, thereby deploying and opening pouch assembly 260. Tube 180 is preferably from a metal such as stainless steel and is preferably coated with a shrink-wrap plastic such as shrinkable polyethylene fiberglass, or polyvinyl chloride of a grade suitable for use in surgical procedures.

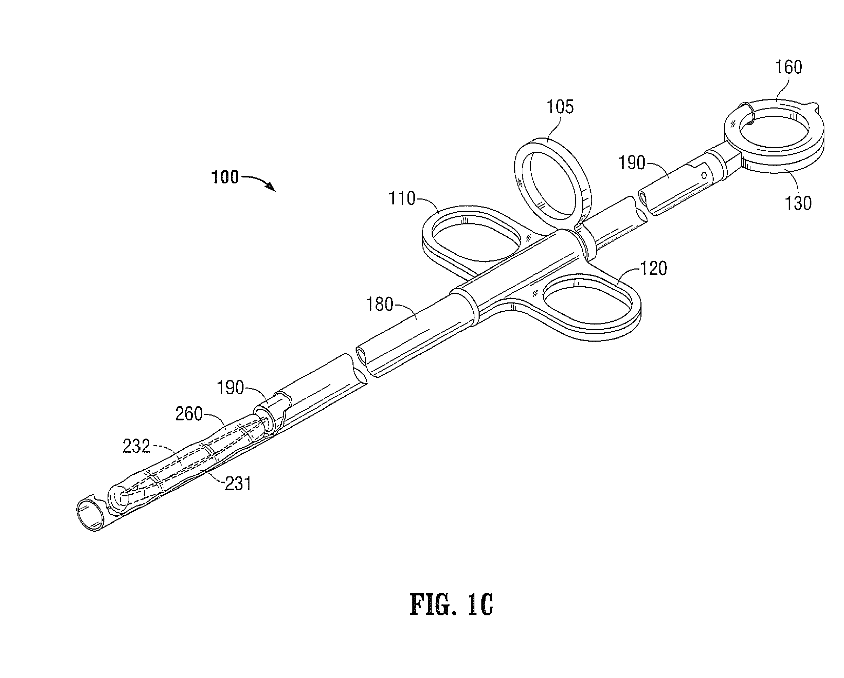

The applicator assembly 100 may include a drive rod or bar 190 that is an elongated generally cylindrical member slidably disposed through the bore of tube 180. A distal end of the drive rod 190 is attached to the pouch assembly 260 to move the pouch assembly 260 from a non-deployed position contained within the outer tube 180 (as shown in FIG. 1C) to a deployed position distal to the outer tube 180, (as shown in FIG. 1A). The drive rod 190 also includes O-rings 210a, 210b, and 210c. The O-rings help maintain a gaseous seal and/or help to maintain the drawstring in place while permitting sliding movement of the drive rod 190 through tube 180.

The drive rod 190 is preferably fabricated from a strong polymeric material. A material suitable for fabricating the drive rod 190 is polycarbonate plastic with 20% glass fiber filler. If gamma sterilization is desired, this material has the additional advantage of being gamma stable. Other materials suitable for the purposes discussed herein may also be used. To maintain a gaseous seal within the instrument, close tolerances are observed. The outer diameter of the drive rod 190 is slightly less than the inner diameter of the tube 180 through which it slides longitudinally. Additionally, the drive rod 190 is preferably coated with a biocompatible lubricant as a viscous sealing material to insure that no gases exit or enter the body through the seal when the operation site (e.g. the peritoneum or other body cavity) is insufflated. Any biocompatible lubricant that will operate as a viscous sealing material may be used, but if gamma sterilization is desired the biocompatible lubricant chosen should be gamma stable. A locking tab 105 is included to prevent premature actuation of the instrument during shipping. The locking tab includes snap fit engagement structure to engage a slot of the drive rod 190. When thus engaged, the drive rod 190 cannot be pushed distally beyond the point where the locking tab 105 engages the proximal end of handle portions 110, 120. To actuate the instrument the surgeon must first disengage the locking tab by pulling it off the instrument.

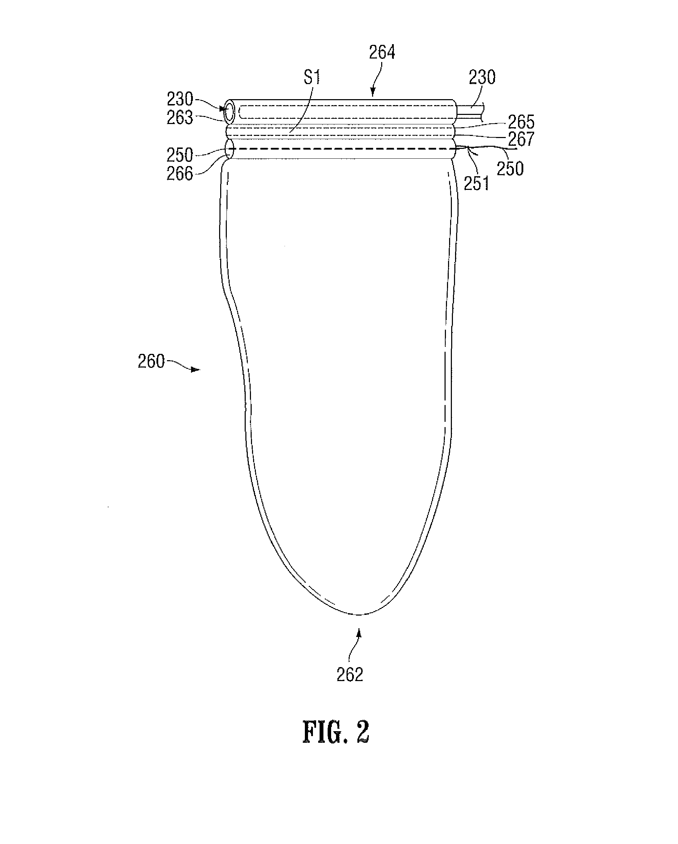

In addition, the applicator assembly 100 may include a finger loop 130 for engagement by a user's finger. One end of a drawstring 250 is attached to the finger loop 130, as shown in FIGS. 1A and 1B while an opposing end of the drawstring 250 is attached to the pouch assembly 260 (see FIG. 2).

Referring now to FIG. 2, in some embodiments, the removal pouch assembly 260 includes a flexible film or sheet preferably formed from a substantially transparent polymeric material. One embodiment of a material is polyurethane sheet, although other biocompatible materials capable of forming a flexible membrane, such as latex, may be used. The material may have a thickness selected be between about 0.001 to about 0.005 inches, although other ranges of thickness may be used as appropriate. The material may be transparent to permit viewing the contents of the pouch assembly 260. The pouch assembly 260 may be formed from an aromatic polyester type thermoplastic polyurethane such as Dureflex®, a product of Deerfield Urethane, Inc. in Whately, Massackhusetts. In addition, the sack material may be impervious or resistant to penetration by cancer cells.

The pouch assembly 260 may be of any dimensions suitable for the purpose of organ entrapment or removal. In the present embodiment, the pouch assembly 260 has a diameter of from about 1.5 inches to about 6.0 inches, a depth of from about 2 inches to about 10 inches, and has a cubic capacity of up to about 2.0 liters of water, depending upon the dimensions of the pouch assembly 260. Pouch assembly 260 includes a closed distal end portion 262 and an openable and closable end portion or mouth 264.

The pouch assembly 260 may alternatively include a circumferential concave portion 263 in the vicinity of the open proximal end portion or mouth 264, for facilitating rolling and placement of the pouch assembly 260 within the elongated tube 180 (See FIG. 1C). The open proximal end portion or mouth 264 is defined by a proximal (upper) circumferential tubular portion or sleeve 263, and a distal (lower) circumferential tubular portion or sleeve 266, which are spaced apart from each other.

The pouch assembly 260 may possess a linear portion 265 weakened by perforation or, more preferably, scoring, which extends circumferentially around the mouth 264 of the pouch assembly between the proximal and distal sleeves 263 and 266, respectively. The scored line 265 may be created by induction heating to create a linear portion having thickness less than that of the original material to facilitate tearing of the material along the scored line 265.

In some embodiments, the pouch assembly 260 includes a seal S1 formed from an elastic material. The scored line 265 defines a proximal (upper) boundary of the seal S1. The height of the seal S1 is defined between the scored line 265 and a distal (lower) boundary 267. The seal S1 has an open configuration and a closed configuration where the elastic material biases the seal S1 to its closed configuration. As the spring exits the distal end of the applicator assembly 100, it expands to open the pouch assembly 260. The expansion of the spring 230 overcomes the bias of the elastic material to simultaneously open the mouth 264 and move the seal S1 to its open configuration.

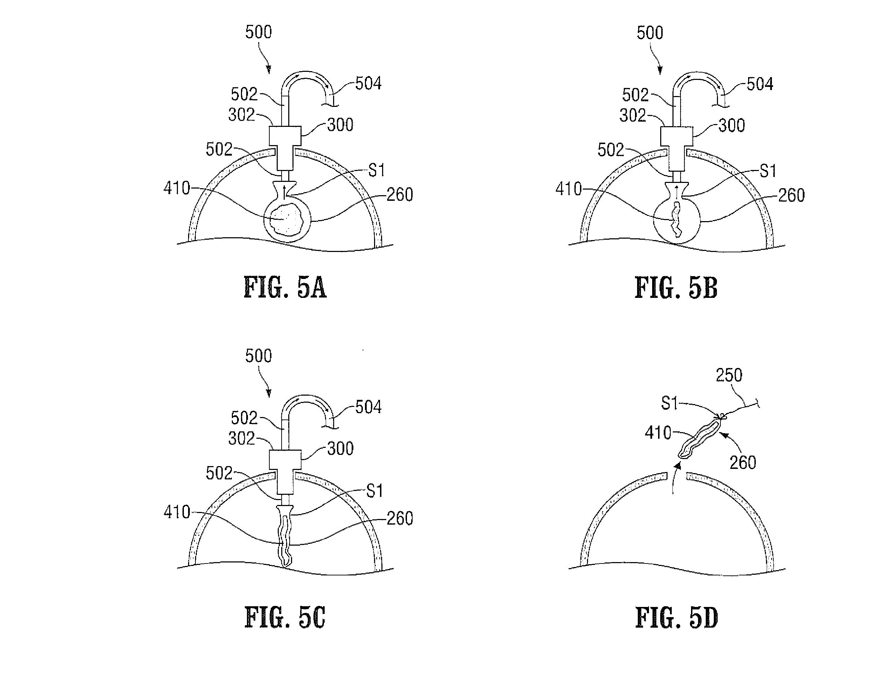

In its open configuration, the seal S1 has substantially the same diameter as the mouth 264. It is preferred that the dimensions of the seal S1, the selected elastic material, and the pouch assembly 260 cooperate with each other such that the seal S1 will form a substantially fluid-tight barrier around a suitably sized surgical instrument (i.e. a suction tube or a morcellator) as illustrated in FIGS. 5A-C. The selected surgical instrument is configured and dimensioned to access an interior portion of a body through a suitably sized access device. It is preferred that the access device has a diameter of 10mm or 15mm, although other suitable diameters are contemplated. By forming a fluid-tight barrier around the surgical instrument, the introduction or escape of insufflation or other gases is minimized. In addition, the fluid-tight barrier minimizes the unwanted entry or escape of solids or liquids from the pouch assembly 260.

The proximal sleeve 263 may be adapted to receive the spring 230, described below. The distal sleeve 266 is adapted to receive the drawstring 250. The scored line 265 is adapted to tear when the drawstring 250 is pulled with sufficient force to close the mouth 264 of the bag distal to the scored line 265, thereby providing fast detachment of pouch assembly 260 from the spring 230 simultaneously with closure of mouth 264. Clearly, alternative structures also can be utilized to detach the pouch assembly 260 from the spring 230, such as by pulling with a grasper or by cutting with a scissors. Simultaneous with the separation of the pouch assembly 260 from the spring 230, the bias of the elastic material of the seal S1 causes the seal S1 to move from its open configuration to its closed configuration.

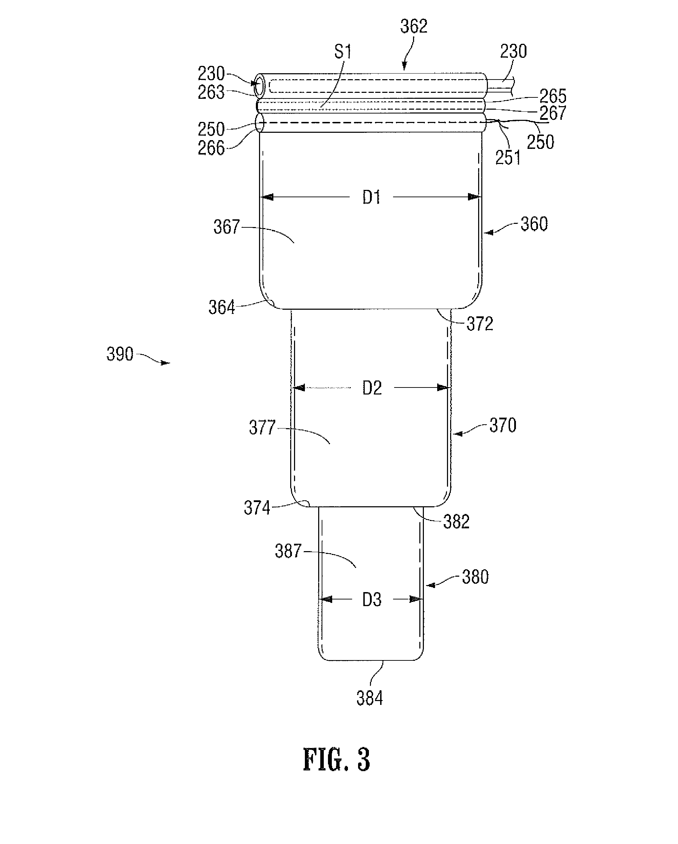

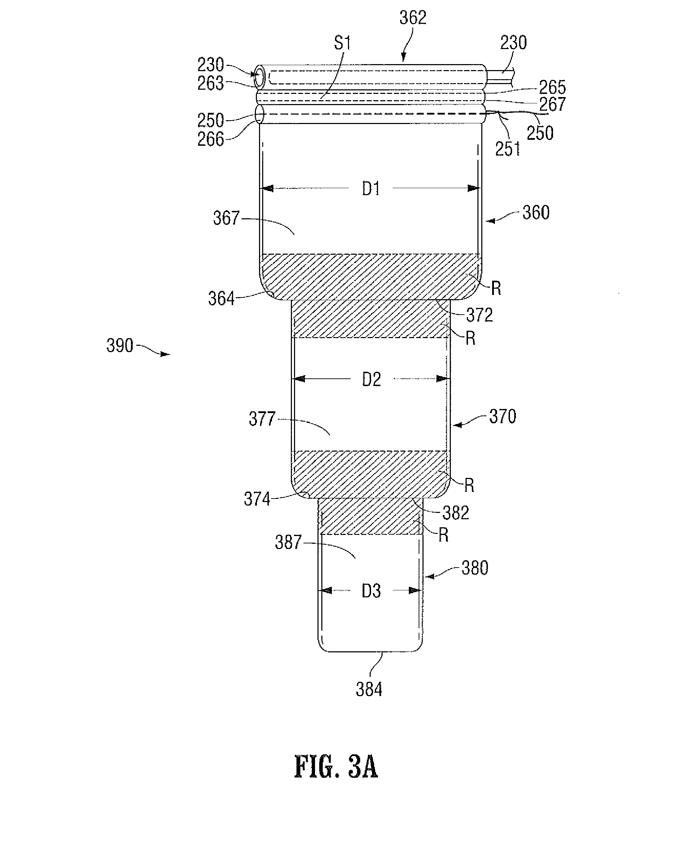

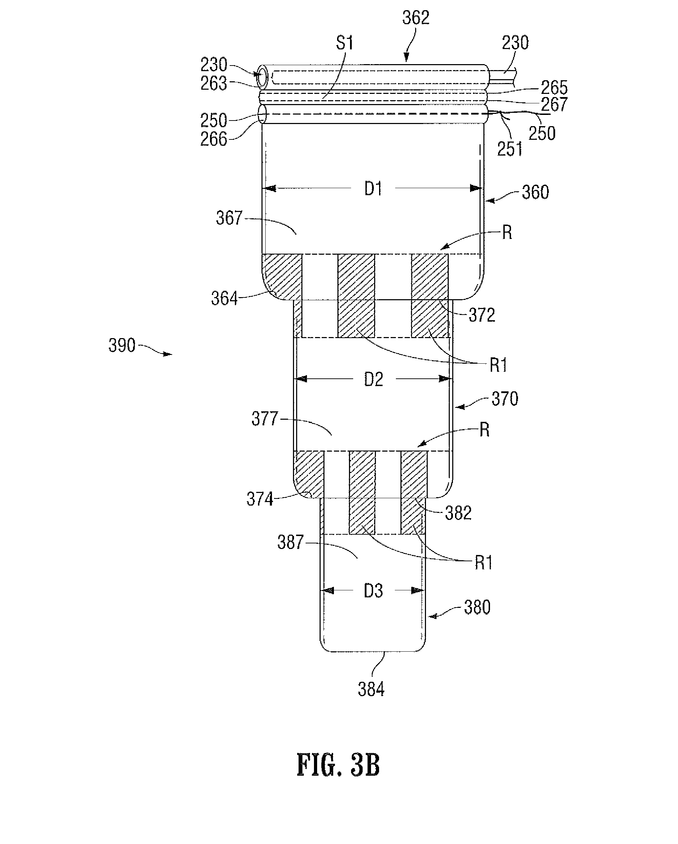

Referring to FIG. 3, an alternate embodiment of the removal pouch assembly or pouch assembly 390 includes a first sack 360, a second sack 370, and a third sack 380. Each sack is formed from a suitable material as discussed in the embodiment of FIG. 2. In addition, the pouch assembly 390 may be of any dimensions suitable for the purpose of organ entrapment or removal as discussed in the embodiment of FIG. 2.

The first sack 360 includes a mouth 362 and an orifice 364 at opposing ends defining a throat 367 therebetween. Preferably, the throat 367 has a diameter D1 that is substantially uniform from the mouth 362 to the orifice 364. Alternately, the first sack 360 may be tapered from the mouth 362 to the orifice 364 forming a frustoconical or an inverted frustoconical shape. In addition, other shapes and configurations of the sack are contemplated. The mouth 362 has an open and a closed configuration while the orifice 364 only has an open configuration.

In particular, the first sack 360 possesses a linear portion weakened by perforation or, more preferably, scoring, which extends circumferentially around the mouth 362 of the first sack 360 between proximal and distal sleeves 263 and 266, respectively. A scored line 265 may be created by induction heating to create a linear portion having thickness less than that of the original material to facilitate tearing of the material along the scored line 265.

Similar to the embodiment illustrated in FIG. 2, the pouch assembly 390 may be adapted to be attached to the spring 230 using the distal sleeve 266 and includes substantially identical structures for the attachment and separation of the pouch assembly 390 of the previous embodiment with the resulting advantages discussed previously.

Still referring to FIG. 3, the second sack 370 has a mouth 372 and an orifice 374 at opposing ends defining a throat 377 therebetween. Similar to the first sack 360, the second sack 370 has a diameter D2 that is substantially uniform from the mouth 372 to the orifice 374. Alternately, the second sack 370 may be tapered from the mouth 372 to the orifice 374 forming a frustoconical or an inverted frustoconical shape. In addition, other shapes and configurations of the sack are contemplated. The mouth 372 is open and in communication with the throat 367 of the first sack 360. It is preferred that the diameter D2 or the diameter of the mouth 372 is less than the diameter D1 or the diameter of the orifice 364. Configured thusly, the throats 367, 377 of the first and second sacks 360, 370, respectively, are in fluid communication with each other and form a staggered arrangement of the sacks included in the pouch assembly 390.

The third sack 380 includes a mouth 382 and a base 384 at opposing ends. The mouth 382 is open while the base 384 is closed defining a cavity 387 therein. The cavity has a diameter D3 that is preferably uniform throughout. Alternately, the third sack 380 may be tapered from the mouth 382 to the base 384 forming a frustoconical or an inverted frustoconical shape. In addition, other shapes and configurations of the sack are contemplated. The mouth 382 is in fluid communication with the throat 377 of the second sack 370. It is preferred that the diameter D3 or the diameter of the mouth 382 is less than the diameter D2 or the diameter of the orifice 374. In this configuration, the throats 367, 377, and 387 are in fluid communication with one another to form a staggered arrangement of the pouch assembly 390.

Referring now to FIG. 3A, an alternate embodiment of the pouch assembly 390 is disclosed. The pouch assembly 390 according to this embodiment includes the same or similar components as the embodiment shown in FIG. 3 and further includes a reinforced band or region R. The reinforced region R overlaps the junction between an orifice and a mouth of a pair of adjacent sacks (e.g. orifice 364 of sack 360 and mouth 372 of sack 370). The dimensions (i.e. thickness and/or height) of the reinforced region R may be influenced by a number of factors including, but not limited to, dimensions of the pouch assembly 390, dimensions of the adjoining sacks, and the task being performed. The reinforced region R improves the overall rigidity of the pouch assembly 390 and helps maintain the staggered or stepped shape of the pouch assembly 390. Additionally, the reinforced regions R improve the strength of the joint between the adjacent sacks thereby minimizing the possibility that the sacks will separate during a surgical procedure or increasing the size and/or mass of the tissue sample to be collected.

In some embodiments, the reinforced region R extends circumferentially about the pouch assembly 390 although it is contemplated that it may only extend for a portion of a circumference of the pouch assembly 390. Alternatively, as illustrated in FIG. 3B, the reinforced region R may include a plurality of reinforced sections R1 that are circumferentially spaced apart forming gaps therebetween. Each reinforced section R1 may have substantially identical dimensions (i.e. thickness, height, and width), although it is contemplated that the dimensions of each of the reinforced sections R1 may be varied for the same or similar reasons discussed for the embodiment of FIG. 3A. As in the previous embodiment, the reinforced region R may extend circumferentially about the pouch assembly 390 or only for a portion thereof. In either of the embodiments of FIGS. 3A or 3B, the reinforced region R may be included in some or all of the pairs of adjacent sacks.

Alternatively, the pouch assembly may only include two sacks where the second sack has a closed end opposite its mouth defining a cavity therein. In other embodiments of the disclosure, additional sacks may be included with the last or most distal sack having a closed end opposite its mouth to define a cavity therein. These alternative configurations increase the flexibility and utility of the pouch assembly 390 of the present disclosure. The pouch assembly may be formed from discrete sacks where the sacks are bonded or joined together using known methods such that each bond is substantially fluid-tight. Alternatively, the pouch assembly may be monolithically formed using known methods to create the staggered arrangement of the included sacks. The pouch assembly of these alternate embodiments may include reinforced bands or regions as previously discussed.

Referring now to FIGS. 5A-D, there is illustrated a method of using the specimen retrieval apparatus of the present disclosure. A suction apparatus 500 includes a suction tube 502, a tubular member 504, and a suction source (not shown) that is connected to a trocar cannula 300 with a first end of the suction tube 502 extending through an external port 302 of the trocar cannula 300 and into the interior portion. One end of the tubular member 504 is attached to the suction source which provides the suction applied to the pouch assembly 260. In addition, the suction apparatus may include storage for liquid and/or solid matter removed or evacuated from the pouch assembly 260. The suction tube 502 may be flexible, rigid, or semi-rigid as determined by the particular application. Preferably, the external surface of the suction tube 502 forms a fluid-tight seal with an external port 302. The second end of the suction tube 502 is attached to the one end of a tubular member 504 and forms a fluid-tight seal therebetween.

After the tissue sample 410 is placed in the pouch assembly 260 using known techniques, the mouth 264 is positioned around the first end of the suction tube 502. The pouch assembly 260 is then separated from the spring 230 by pulling the drawstring 250 in a proximal direction. This attaches the pouch assembly 260 and its contents to the suction tube 502. As discussed previously with respect to FIG. 2, the pouch assembly 260 separates from the spring 230 and the seal S1 moves to its closed configuration and forms a substantially fluid-tight barrier around the external surface of the suction tube 502 with the attendant advantages previously discussed.

After the pouch assembly 260 is attached to the suction tube 502, the suction source is actuated and applies suction to the pouch assembly 260. The applied suction is communicated through the tubular member 504 and the suction tube 502 to the pouch assembly 260. The surgical procedure being performed, the characteristics of the tissue sample 410 (i.e. size, mass, density, etc.), and the volume of any liquids present in the pouch assembly 260 may be factors in determining the amount and duration of the applied suction. As illustrated in FIG. 5B, the applied suction to the pouch assembly 260 concurrently evacuates liquids and particulate matter (i.e. portions of the tissue sample 410 or other particulate matter) from the pouch assembly 260.

Since seal S1 forms a substantially fluid-tight barrier around the external surface of the suction tube 502, as suction is applied to the pouch assembly 260 to remove or evacuate the particulate matter and/or liquid from the pouch assembly 260, the flexible membrane collapses to conform the pouch assembly 260 to the shape and configuration of the tissue sample 410 as shown in FIG. 5C. The fluid-tight barrier formed by seal S1 minimizes the introduction of insufflation gases into the pouch assembly 260 thereby allowing the applied suction to act only on the contents of the pouch assembly without negatively affecting the insufflation pressure in the interior portion.

Once the particulate matter and/or liquid have been evacuated and the pouch assembly 260 has been reduced to a desired size, the applied suction may be discontinued or reduced. By reducing the volume of the pouch assembly 260 and its contents, improved retrieval of tissue specimens is obtained (see FIG. 5D). Further still, larger tissue samples may be retrieved for a particular size of trocar cannula using the device and methods discussed hereinabove as compared to conventional devices and methods for retrieval. Although the above discusses one embodiment of the pouch assembly, the techniques discussed are equally applicable to other embodiments of the pouch assembly previously disclosed herein.

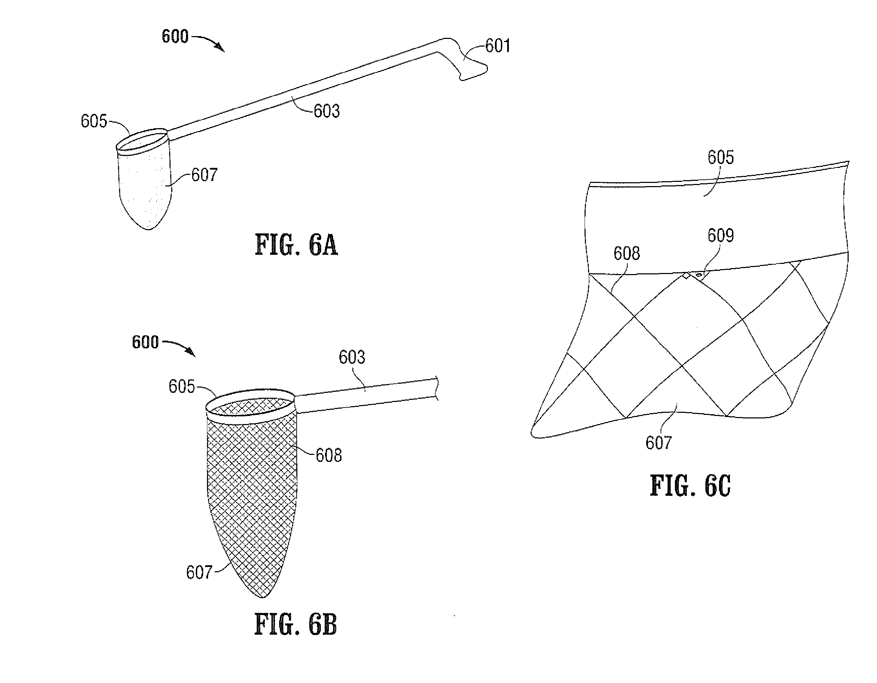

In accordance with at least one aspect of the present disclosure, a specimen retrieval device 600 is described herein. Referring to FIGS. 6A, 6B, and 6C, an embodiment of the specimen retrieval device 600 is shown including a handle 601, a shaft 603, a bag deployment device 605, and a specimen retrieval bag 607. The specimen retrieval device 600 may be substantially similar to the applicator assembly 100 as described above.

The handle 601 may be shaped in any desirable way and include any triggers, buttons, or effectors for operating any devices associated with the bag deployment device 605 as described herein. The handle 601 may be substantially similar to handle portions 110, 120 as described above.

The shaft 603 may be connected, either integrally or removably, to the handle and extend distally therefrom. The shaft 603 may be made of any rigid or semi rigid material, including but not limited to at least one of a biocompatible metal or alloy, plastic, or polymer. The shaft 603 may further be selectively flexible or deformable. The shaft 603 may be substantially similar to tube 180 as described above, and may further include any and all functional elements for removal pouch deployment as described herein.

The bag deployment device 605 is operably connected to a distal end of the shaft 603, either integrally or removably. The bag deployment device 605 may be a fixed frame for the specimen retrieval bag to fixedly connect to, and may be made of any rigid or semi rigid material. The bag deployment device 605 may further include a system for collapsing to create a smaller profile for insertion and removal. The bag deployment device 605 may further include a system for tightening, tying off, or sealing the specimen retrieval bag 607 for specimen removal. In some embodiments, the bag deployment device 605 is substantially similar to spring 230 as described above.

The specimen retrieval bag 607 is connected to the bag deployment device 605 and includes at least one material having a mesh 608 disposed therein or thereon. The at least one material may be any flexible material including but not limited to one or more of a plastic, fabric, polymer, or rubber. In some embodiments, the material is at least one fabric including the mesh 608 disposed therein. The at least one fabric may be rip-stop nylon. The bag 607 may be fixedly or operably connected or attached to the bag deployment device 605 as described herein. Specimen retrieval bag 607 may be substantially similar to the removal pouch assembly 260, 360 as described above.

The mesh 608 may include any rigid or semi-rigid material such as but not limited to one or more of a metal, plastic, polymer, etc. The mesh 608 may be geometrically arranged as desired such that the mesh 608 provides different flexibilities as a function of the mesh 609 rotation relative to a direction of force. For example, as shown in the figures, the mesh 609 may be arranged as a series of parallel wires or threads intersecting at approximately a right angle with another series of parallel wires or threads to create a series of boxes or ladders.

The mesh 608 is disposed at an angle 609 relative to the bag deployment device 605. The angle 609 is acute, such that it is not equal to 90 degrees. The angle 609 may be about 45 degrees. The angle 609 may be between about 1 and about 45 degrees. Theoretically, the angle 609 may range from any angle between 0 degrees and 360 degrees, however, for a mesh 608 configuration as shown, all relative variations may be made using an angle 609 of 90 degrees or less.

The purpose for angling the mesh 609 relative to the bag deployment device 605 is that the flexibility of the bag is selectable as a function of the angle 609. The mesh 609 may have a minimum flexibility when the angle 609 is a first value and a maximum flexibility when the angle 609 is at a second value. For example, the mesh 609 as shown in the figures has a minimum flexibility when the angle 609 is about 90 degrees and a maximum flexibility when the angle 609 is about 45 degrees. When the specimen retrieval bag 607 is being removed from a patient, the specimen retrieval bag 607 often has to travel through a tight space or lumen causing the specimen retrieval bag 607 to be subjected to a pulling force. The more the specimen retrieval bag 607 is allowed to flex in such a situation, the easier it can fit through tight spaces without causing tissue damage and allow the contents of the bag 607 to stretch and thin out as a result of the flex. Thus, by using a specimen retrieval bag 607 having a mesh 608 as described above, a desired level of flexibility may be selected, reducing the required force to pull the specimen retrieval bag 607 through constricted spaces and reducing the likelihood of incident tissue damage.

In at least one embodiment, the specimen retrieval bag 607 includes rip-stop nylon including a rip-stop mesh that is angled about 45 degrees relative to the bag deployment device 605.

In accordance with still yet another aspect of this disclosure, a method for modifying flexibility of a specimen retrieval bag 607 includes providing a specimen retrieval device 600, as described above, and selecting the angle 609 from a range of about 90 degrees to about 45 degrees to provide a desired flexibility of the specimen retrieval bag 607 between a range of flexibilities, the range of flexibilities being from a minimum flexibility when the angle 609 is about 90 degrees to a maximum flexibility when the angle is about 45 degrees.

The angle 609 may be selected to be about 45 degrees for maximum flexibility of the specimen retrieval bag 607. The angle 609 may be selected to be about 30 degrees. The angle 609 may be selected to be about 20 degrees.

In some embodiments, the material is rip-stop nylon and the angle 609 is selected to be about 45 degrees for maximum flexibility of the specimen retrieval bag 607.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figs. are presented only to demonstrate certain examples of the disclosure. Other elements, steps, methods and techniques that are insubstantially different from those described above and/or in the appended claims are also intended to be within the scope of the present disclosure.

## Claims

1. A specimen retrieval device, comprising:
a handle;
a shaft connected to said handle and extending distally;
a bag deployment device connected to a distal end of the shaft; and
a specimen retrieval bag connected to the bag deployment device and including at least one material having a mesh disposed therein, at least one strand of the mesh being disposed at an angle relative to the bag deployment device, wherein the angle is acute, wherein the flexibility of the bag is selectable as a function of the angle.

2. The specimen retrieval device of claim 1, wherein the angle is about 45 degrees.

3. The specimen retrieval device of claim 1, wherein the angle is between about 1 and about 45 degrees.

4. The specimen retrieval device of claim 1, wherein the material is at least one fabric including the mesh disposed therein.

5. The specimen retrieval device of claim 4, wherein the at least one fabric is rip-stop nylon.

6. The specimen retrieval device of claim 5, wherein the angle is about 45 degrees.

7. A method for modifying flexibility of a specimen retrieval bag, comprising:
providing a specimen retrieval device including:
a handle;
a shaft connected to said handle and extending distally;
a bag deployment device connected to a distal end of the shaft; and
a specimen retrieval bag connected to the bag deployment device and having at least one material having a mesh disposed therein, at least one strand of the mesh being disposed at an angle relative to the bag deployment device, wherein the flexibility of the bag is selectable as a function of the angle; and
selecting the angle from a range of about 90 degrees to about 45 degrees to provide a desired flexibility of the specimen retrieval bag between a range of flexibilities, the range of flexibilities being from a minimum flexibility wherein the angle is about 90 degrees to a maximum flexibility wherein the angle is about 45 degrees.

8. The method of claim 7, wherein the angle is selected to be about 45 degrees for maximum flexibility of the specimen retrieval bag.

9. The method of claim 7, wherein the angle is selected to be about 30 degrees.

10. The method of claim 7, wherein the angle is selected to be about 20 degrees.

11. The method of claim 7, wherein the material is rip-stop nylon and the angle is selected to be about 45 degrees for maximum flexibility of the specimen retrieval bag.

12. A specimen retrieval device, comprising:
a handle;
a shaft connected to said handle and extending distally;
a bag deployment device connected to a distal end of the shaft; and
a specimen retrieval bag connected to the bag deployment device and having rip-stop nylon having a rip-stop mesh disposed therein, at least one strand of the rip-stop mesh being disposed at a 45 degree angle relative to the bag deployment device.
